# FASCICULE DE BREVET EUROPEEN

(11) **EP 1 563 800 B1**
(45) Date de publication et mention de la délivrance du brevet: **13.02.2008**
(21) Numéro de dépôt: 05001994.2
(22) Date de dépôt: 01.02.2005
(51) Int. Cl.: A61C 1/14, A61B 17/16, B23B 31/20

(54) **Pièce à main à usage dentaire ou chirurgical**
Dentalmedizinisches oder chirurgisches Handstück
Dental or surgical handpiece

(30) Priorité: 17.02.2004 EP 04003487
(43) Date de publication de la demande: 17.08.2005
(73) Titulaire: Bien-Air Holding SA, 2500 Bienne 6 (CH)
(72) Inventeur: Maître, Luc, 2885 Epauvillers (CH)
(74) Mandataire: Laurent, Jean

(56) Documents cités:
- US-A- 3 631 597
- US-A- 4 260 381

## Description

### Arrière-plan de l'invention

La présente invention concerne une pièce à main à usage dentaire ou chirurgical, du genre comportant : un arbre creux rotatif autour d'un axe longitudinal et monté par des roulements dans un fourreau tubulaire fixe; une pince montée dans une extrémité avant de l'arbre creux et ayant un canal central destiné à recevoir la queue d'un outil amovible, la pince comportant des branches axiales réparties autour de l'axe et pourvues chacune d'un mors dans le canal central, chaque branche étant rattachée à une partie annulaire de la pince; un mécanisme de serrage porté par l'arbre creux et agencé pour exercer une force centripète sur une surface d'appui de chaque branche de la pince pour serrer la queue de l'outil entre les mors de la pince; et un mécanisme de desserrage porté au moins en partie par le fourreau fixe et agencé pour agir sur le mécanisme de serrage au moins de façon à relâcher la pince.

Habituellement, dans les pièces à main de ce genre, les mors sont situés à une extrémité de la pince qui présente des surfaces extérieures d'appui de forme conique, sur lesquelles s'engage un cône intérieur d'une pièce tubulaire qui serre ainsi l'extrémité de la pince sur la queue de l'outil. Par exemple, selon la demande de brevet FR 2 191 869, correspondant au brevet US 3 902 248, la pince est montée de manière coulissante dans l'arbre creux, ses mors et ses surfaces extérieures d'appui se trouvent à son extrémité avant pour coopérer avec l'orifice avant de l'arbre, et la pince est maintenue serrée par un ressort de serrage qui la tire axialement vers l'arrière.

Dans les cas où l'outil porté par la pièce à main doit supporter des efforts relativement élevés, notamment en ce qui concerne le couple et les forces axiales, le serrage de la pince doit être assez fort. Cela signifie une force axiale assez élevée du ressort de serrage, ainsi que des forces de frottement élevées sur les surfaces coniques qui transforment cette force axiale en forces radiales de serrage. Le mécanisme de desserrage doit pouvoir surmonter ces forces en appliquant une poussée axiale suffisant à l'organe coulissant sollicité par le ressort de serrage. Cette poussée axiale est alors transmise au bâti de la pièce à main à travers au moins l'un des roulements supportant l'arbre rotatif. La plupart du temps, comme dans la construction selon la publication FR 2 191 869, cette poussée s'exerce vers l'avant et charge donc les roulements dans le sens opposé aux charges axiales que l'outil subit, c'est-à-dire que les roulements doivent être conçus pour supporter des charges axiales dans les deux sens.

En outre, un ressort de serrage relativement fort a une masse relativement élevée, ce qui constitue un inconvénient notable pour une pièce qui tourne très vite et dont le centrage ne peut généralement pas être assuré avec précision. Les vibrations qui en résultent augmentent avec la masse de ce ressort.

La demande de brevet FR 2 723 306, correspondant au brevet US 5 688 122, décrit un dispositif de serrage d'un outil dentaire dans une tête de pièce à main coudée, comportant une pince ayant des branches élastiques formées par des découpes dans une douille servant aussi de canon de guidage de la queue de l'outil. Les mors situés sur les branches de la pince sont, en position de repos, à une distance de l'axe inférieure au rayon de la queue de l'outil, de sorte que celle-ci est ensuite serrée uniquement par l'élasticité des branches. Ce dispositif se passe d'un ressort axial de serrage, mais en revanche la force centrifuge tend à desserrer la pince.

Le brevet US 4 260 381 décrit un dispositif de serrage d'un outil dentaire comportant une pince montée à l'extrémité d'un arbre creux.

### Résumé de l'invention

La présente invention, telle que définie dans la revendication 1, vise à éviter les inconvénients susmentionnés de l'art antérieur, grâce à un agencement qui assure un fort serrage au niveau des mors de la pince tout en limitant les forces axiales intervenant dans les mécanismes de serrage et de desserrage. Un but additionnel de l'invention est de simplifier l'agencement des roulements des paliers de la pièce à main, en particulier en réduisant les charges axiales sur au moins l'un d'eux.

A cet effet, il est prévu une pièce à main du genre indiqué en préambule ci-dessus, caractérisée en ce que chaque branche de la pince comprend un levier dont une première extrémité est rattachée à la partie annulaire par une articulation et dont ladite surface d'appui est distante axialement de cette articulation, le mors étant situé axialement plus près de l'articulation que de la surface d'appui.

Ainsi, par effet de levier, la force de serrage de chaque mors est supérieure au double de la force centripète appliquée sur la surface d'appui par le mécanisme de serrage, et en pratique le rapport des bras du levier peut s'élever aisément jusqu'à dix ou plus, car la course de serrage au niveau des mors peut être très petite.

Le mécanisme de serrage peut comporter un coulisseau monté dans l'arbre creux, pourvu d'une surface conique concave du côté de la pince et sollicité axialement par un ressort qui presse sa surface conique contre lesdites surfaces d'appui des leviers pour maintenir la pince serrée. Si l'inclinaison des surfaces d'appui par rapport à l'axe est faible. Il en résulte encore une multiplication de l'effet de la force du ressort sur chaque levier de la pince.

Cependant, selon un mode de réalisation préféré, le mécanisme de serrage comporte un manchon coulissant monté autour de l'arbre creux et, pour chaque levier de la pince, un élément de transmission disposé dans un trou radial de l'arbre creux entre ladite surface d'appui du levier et une surface de came intérieure du manchon coulissant, la surface de came comportant une surface inclinée axialement telle qu'une surface conique. Ce manchon coulissant peut être sollicité dans le sens du serrage par un ressort relativement faible. Dans une variante particulièrement avantageuse, on peut ménager dans la surface de came un creux formant un cran qui maintient en place le manchon coulissant par l'élasticité des leviers de la pince, ce qui permet de supprimer le ressort de serrage habituel. En conséquence, la manoeuvre de desserrage n'a plus à surmonter la poussée axiale due à ce ressort.

D'autres caractéristiques et avantages de la présente invention apparaîtront dans la description suivante de différents modes de réalisation, présentés à titre d'exemples non limitatifs en référence aux dessins annexés.

### Description sommaire des dessins

La figure 1 est une vue schématique en perspective d'une pièce à main selon l'invention.

La figure 2, divisée en deux fractions 2(a) et 2(b), est une vue en coupe longitudinale d'un premier mode de réalisation de la pièce à main de la figure 1, comportant une pince associée à un mécanisme de serrage à billes.

Les figures 3 à 5 représentent la pince visible dans la figure 2, respectivement en perspective, en coupe longitudinale et en vue latérale.

La figure 6 est une vue partielle en perspective d'un dispositif de commande de serrage de la pince.

La figure 7 est une vue de l'accouplement entre les deux arbres de la pièce à main.

La figure 8 représente une pièce intercalaire de l'accouplement.

La figure 9 est une vue de détail en coupe longitudinale représentant un deuxième mode de réalisation, grâce à une modification du mécanisme de serrage à billes visible dans la figure 2.

La figure 10 est une vue en coupe longitudinale partielle d'un troisième mode de réalisation de la pièce à main de la figure 1, comportant une pince associée à un mécanisme de serrage à cône.

Les figures 11 et 12 représentent la pince visible dans la figure 10, respectivement en perspective et en coupe longitudinale.

Les figures 13 et 14 représentent une variante de la pince visible dans la figure 10, respectivement en perspective et en coupe longitudinale.

Les figures 15 et 16 représentent, respectivement en coupe longitudinale et en perspective, une douille additionnelle pouvant être combinée à la pince de la pièce à main.

### Description détaillée de modes de réalisation de l'invention

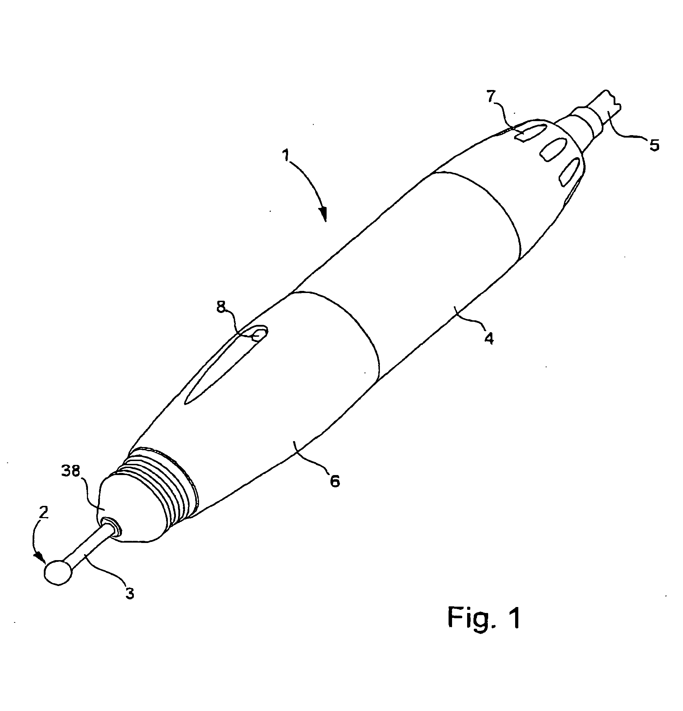

La figure 1 représente une pièce à main 1 à usage dentaire ou chirurgical, dans laquelle on peut trouver les divers modes de réalisation de l'invention qui seront décrits plus loin. La pièce à main est équipée d'un outil rotatif amovible 2 ayant une queue cylindrique 3 qui est fixée par serrage dans une pince rotative de la pièce à main 1. Celle-ci contient un moteur, en l'occurrence un moteur électrique, pour faire tourner l'outil 2 à grande vitesse. Le moteur est logé dans le corps principal 4 de la pièce à main et il est alimenté et commandé à partir d'une unité extérieure via un câble électrique 5 raccordé à l'arrière de la pièce à main. L'opérateur commande le serrage et le desserrage de la pince en faisant tourner dans un sens ou dans l'autre un manchon 6 monté de manière rotative sur le corps 4. Les références 7 et 8 désignent des ouies de ventilation. Un tel instrument trouve ses applications notamment dans les cabinets dentaires, dans les laboratoires dentaires et dans les techniques de microchirurgie. Dans les exemples représentés ici, il s'agit d'un instrument pour laboratoire dentaire, utilisant des outils dont la queue a un diamètre normalisé de 2,35 mm.

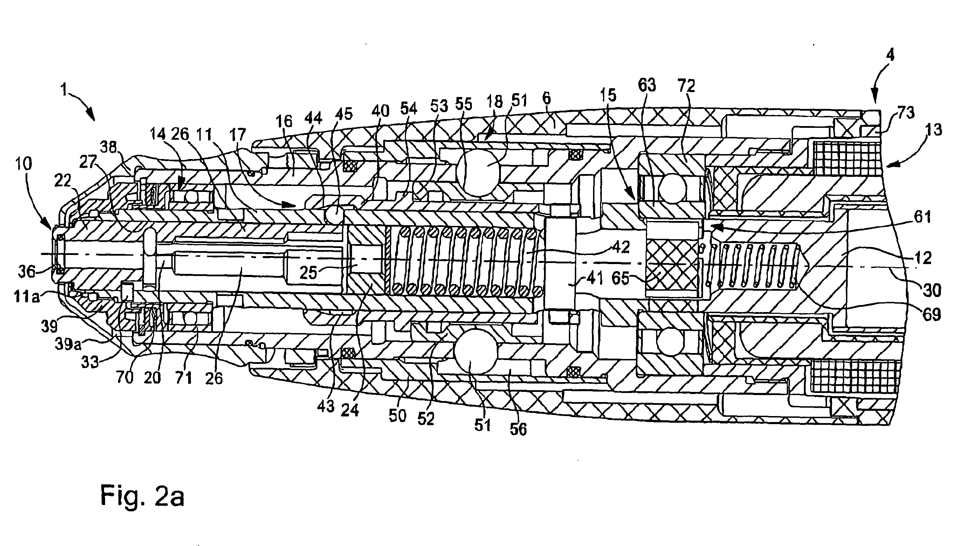

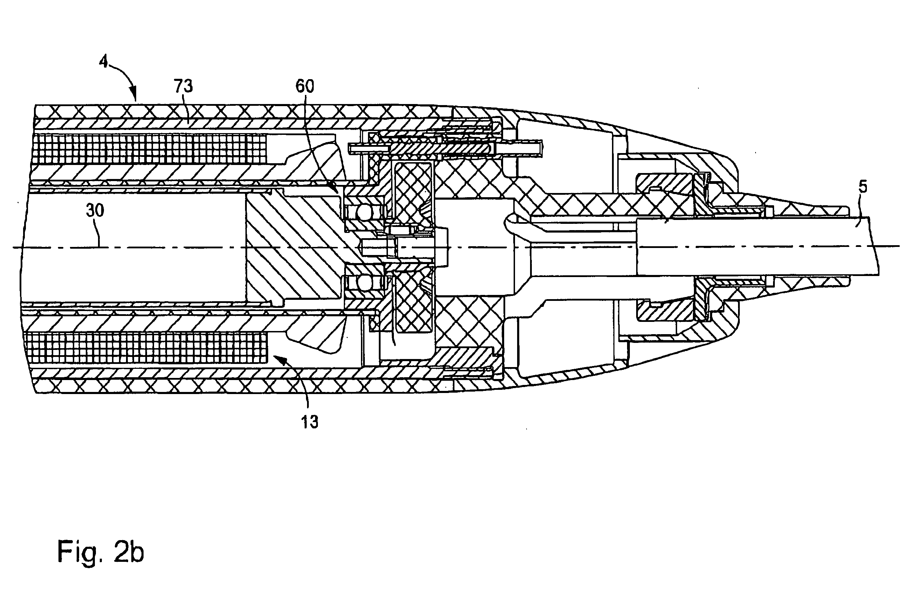

On décrira maintenant, en référence aux figures 2 à 5, un premier mode de réalisation de la pince de serrage 10 de l'outil et des mécanismes de serrage et de desserrage de cette pince. La pince 10 est située dans l'extrémité avant de la pièce à main, à l'intérieur d'un arbre rotatif creux 11 couplé à l'arbre 12 du moteur électrique 13. L'arbre 11 est supporté par des roulements à billes 14 et 15 dans un fourreau 16 fixé au corps 4 de la pièce à main et il peut ainsi tourner à des vitesses de l'ordre de 50 000 tours/minute dans l'instrument représenté ici. Cependant, une pince de serrage telle que la pince 10 est aussi utilisable dans des instruments dont l'outil peut tourner à plusieurs centaines de milliers de tours par minute, notamment avec un entraînement par turbine à air.

Un mécanisme 17 de serrage de la pince 10 est monté sur l'arbre 11 et tourne avec lui. Un mécanisme 18 de desserrage de la pince, commandé par la rotation du manchon 6, est monté sur le fourreau non rotatif 16 et peut agir sur le mécanisme de serrage 17 afin de libérer l'outil lorsque la rotation est arrêtée. Ces mécanismes seront décrits en détail plus loin.

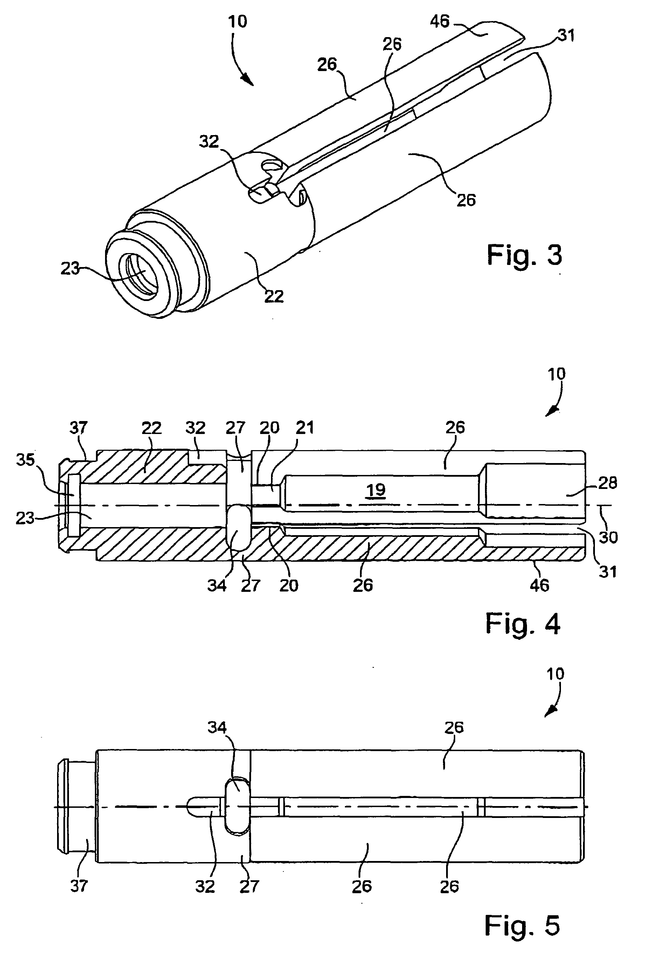

Les figures 3 à 5 montrent plus particulièrement le premier mode de réalisation de la pince 10, qui comporte ici trois mors 20 régulièrement répartis autour d'un canai central 19 de la pince, recevant la queue 3 de l'outil. Chaque mors 20 a une portion de surface cylindrique 21 destinée à s'appliquer contre la queue de l'outil.

A l'avant, la pince 10 comporte un canon d'entrée 22 sensiblement cylindrique, pourvu d'un alésage axial 23 calibré avec une grande précision afin de centrer l'outil aussi parfaitement que possible. On notera dans la figure 2 qu'un canon arrière de guidage 24 est fixé dans l'arbre 11 en arrière de la pince 10 et comporte un alésage central 25 destiné à guider l'extrémité de la queue de l'outil.

Chaque mors 20 fait partie intégrante d'un levier respectif 26 qui s'étend axialement vers l'arrière à partir du canon d'entrée 22, auquel il est rattaché par une partie flexible 27 formant en quelque sorte une articulation sur laquelle le levier 26 peut pivoter en direction de l'axe de rotation 30 de l'arbre. Sur chaque levier 26, le mors 20 se trouve beaucoup plus près de la partie flexible 27 que de l'extrémité libre 28 du levier, de sorte qu'une force radiale appliquée sur le levier près de son extrémité 28 produit une force de serrage très élevée au niveau du mors 20.

La pince 10 représentée dans les dessins est faite de préférence d'une seule pièce métallique, par exemple en acier. Les leviers 26 sont séparés les uns des autres par des fentes axiales 31 se prolongeant chacune par une encoche 32 dans l'arrière du canon 22. Dans chaque encoche 32 s'engage une goupille 33 qui solidarise en rotation la pince 10 et l'arbre creux 11. Des fentes transversales 34 réduisent l'épaisseur des leviers 26 à leur base et définissent ainsi les parties flexibles 27 dans trois régions périphériques de la section transversale de la pince. A l'avant du canon d'entrée 22, il est prévu une rainure annulaire intérieure 35 pour un joint torique 36 et une rainure annulaire extérieure 37 pour des lèvres d'un capuchon fixe 38 et d'un écrou 39 vissé sur l'arbre 11 pour retenir axialement la pince 10. L'écrou 39 est pourvu d'ailettes de ventilation 39a destinées à créer une légère pression d'air sous le capuchon 38 afin de prévenir l'entrée de saletés par la fente entre le capuchon et le canon 22. En outre, l'écrou 39 serre radialement l'extrémité 11a de l'arbre creux 11 contre le canon 22, cette extrémité étant amincie et divisée en languettes flexibles par des fentes axiales. Ceci assure un centrage sans jeu de la pince 10 dans l'arbre creux.

Dans une variante non représentée ici, le canon d'entrée 22 peut être une pièce distincte, ne faisant pas partie de la pince 10. L'avant de celle-ci est alors formé d'une courte partie annulaire à laquelle les leviers 26 sont rattachés par une articulation. Cette partie annulaire peut être une pièce distincte des leviers, selon les besoins, mais un exécution en une pièce est préférable en général.

Le mécanisme de serrage 17 comporte un manchon 40 qui est monté de manière coulissante autour de l'arbre creux 11, avec lequel il est solidarisé en rotation par un barreau transversal 41 engagé dans des fentes longitudinales de l'arbre 11 et du manchon 40. Un ressort de compression 42 prenant appui sur le canon de guidage arrière 24 pousse le barreau 41 axialement vers l'arrière. L'extrémité avant du manchon 40 comporte une gorge intérieure 43 délimitée à l'avant par une surface conique 44. Trois billes 45 sont logées dans des trous correspondants de l'arbre creux 11 et ont un diamètre qui correspond à la distance entre la surface intérieure de l'arbre 11 et le fond de la gorge 43 du manchon 40. Chaque bille 45 s'appuie sur la surface extérieure d'un des leviers 26 de la pince 10, près de l'extrémité 28 du levier.

Quand le manchon 40 est libéré du mécanisme de desserrage 18, il tend à coulisser vers l'arrière sous l'effet de la poussée axiale du ressort 42, de sorte que sa surface conique 44 pousse radialement vers l'intérieur l'extrémité de chaque levier 26 par l'intermédiaire de la bille correspondante 45. Par pivotement du levier sur la partie flexible 27, cette force se transmet d'une manière multipliée sur le mors 20 du levier et serre donc très fortement la queue de l'outil, et ceci de manière permanente pendant le travail. Les gens du métier comprendront qu'avec un tel mécanisme de serrage, les leviers 26 de la pince peuvent être soit rigides, soit légèrement flexibles. S'ils sont rigides, les déplacements radiaux de leurs extrémités sont simplement un peu plus petits, et il en va de même pour le déplacement axial du manchon 40. Dans les deux cas, une grande force de serrage au niveau des mors 20 est maintenue en permanence même si la force du ressort 42 est relativement modeste, grâce à l'effet de levier de la pince et aussi grâce à la faible inclinaison de la surface conique 44 par rapport à l'axe 30. Cette faible inclinaison a aussi pour effet que la force centrifuge agissant sur les leviers 26 ne peut pas surmonter l'effet du ressort 42. Il faut remarquer en outre que la transmission des efforts via les billes 45 s'effectue avec très peu de friction, ce qui contribue aussi à maintenir une force déterminée de serrage. Toutefois ces billes ne sont pas indispensables, car elles pourraient être remplacées par d'autres éléments de transmission traversant l'arbre 11 et agissant sur les leviers 26.

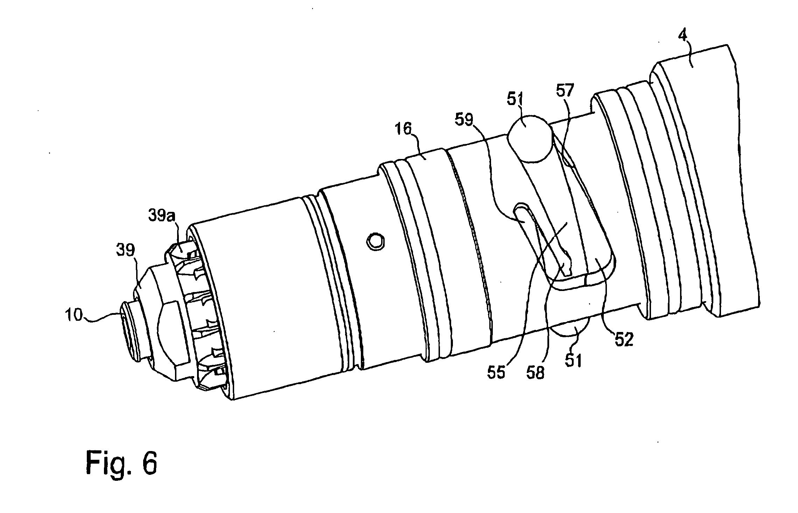

Le mécanisme de desserrage 18 est conçu pour repousser le manchon 40 vers l'avant, contre la force du ressort 42, lorsque l'utilisateur fait tourner le manchon de commande 6 sur le corps 4 dans le sens correspondant. Il comprend une douille 50 liée en rotation au manchon de commande 6, une ou plusieurs billes 51, en l'occurrence deux billes disposées symétriquement par rapport à l'axe 30 de la pièce à main, et une bague de poussée 52 montée de manière coulissante dans le fourreau 16 et ayant à l'avant un rebord intérieur 53 destiné à s'appuyer axialement contre un collet extérieur 54 du manchon 40 lorsque ce dernier ne tourne pas. Les billes 51 sont engagées dans une gorge extérieure annulaire 55 de la bague 52. En outre, chaque bille 51 est engagée dans une rainure axiale correspondante 56 de la douille 50 et dans une fente inclinée 57 (figure 6) du fourreau 16. Le tracé de chaque fente 57 est sensiblement hélicoïdal, pour déterminer un certain déplacement axial de la bille 51, et ses extrémités peuvent être légèrement coudées afin de mieux définir une position d'arrêt de la bille. Dans la forme préférée représentée à la figure 6, l'extrémité arrière de chaque fente 57, correspondant à la position reculée de la bague 52 et donc à un état de serrage de la pince 10, est équipée d'une languette flexible 58 dont l'extrémité a une bosse qui retient par encliquetage la bille 51 au bout de la fente 57. Ceci a pour effet de retenir élastiquement le manchon de commande rotatif 6 afin de prévenir un actionnement intempestif et faire sentir à l'utilisateur qu'il va quitter la position normale de travail de la pince. On remarque que la languette flexible 58 est réalisée simplement par fraisage d'une fente supplémentaire 59 dans le fourreau 16.

Un aspect remarquable de la pièce à main, illustré notamment par la figure 2, est le fait que la partie tournante est supportée par trois paliers seulement, à savoir le roulement à billes avant 14, le roulement à billes central 15 et un roulement à billes arrière 60, les deux arbres coaxiaux 11 et 12 étant supportés tous les deux par le roulement central 15 à l'intérieur duquel ils sont liés en rotation l'un à l'autre par un accouplement positif 61 représenté plus particulièrement dans les figures 2, 7 et 8. L'extrémité arrière de l'arbre creux 11 présente un épaulement 62 qui bute contre la bague intérieure 63 du roulement 15. Elle présente en outre un groupe de crabots 64 qui est chassé dans la bague 63, en l'occurrence trois crabots répartis à 120 degrés les uns des autres sur la circonférence. De son côté, l'extrémité avant de l'arbre 12 du moteur est insérée dans la bague intérieure 63 du roulement 15, de préférence de manière coulissante pour pouvoir y effectuer les petits déplacements axiaux pouvant résulter des dilatations thermiques, des jeux axiaux des roulements et d'autres tolérances. Cette extrémité de l'arbre 12 comporte également des crabots 65 s'étendant axialement entre les crabots 64 de l'autre arbre, pour assurer la transmission du couple dans les deux sens entre les deux arbres. Une pièce intercalaire 66, de préférence en matière synthétique, comporte un corps central cylindrique 67 et des ailettes radiales 68 qui sont intercalées entre les crabots adjacents 64 et 65 pour servir de coussinets. De plus, la pièce intercalaire 66 est précontrainte axialement contre l'arbre creux 11 par un ressort de compression 69 logé dans l'arbre 12 et dont le rôle apparaîtra plus loin.

Le roulement avant 14 est précontraint au moyen d'un ressort diaphragme 70 qui pousse vers l'arrière la bague extérieure 71 du roulement, capable de coulisser dans le fourreau 16. Cette précontrainte axiale se transmet à travers le roulement 14 et l'arbre 11 jusqu'à la région du roulement central 15 où elle se répartit d'une part sur la pièce intercalaire 66 et l'arbre 12 du moteur, dans une mesure égale à la poussée axiale du ressort 69, et pour le reste dans le roulement central 15 sous la forme d'une précontrainte qui retourne au fourreau 16 via la bague extérieure 72 du roulement. La force axiale que le ressort 69 exerce sur l'arbre 12 constitue évidemment la précontrainte axiale du roulement arrière 60. Grâce au jeu axial de l'accouplement 61 entre les deux arbres, cette charge ne varie pas lorsque l'utilisateur exerce une poussée axiale sur l'outil, car cette poussée est entièrement absorbée par le roulement central 15, dont la bague extérieure 72 est épaulée par l'extrémité d'un élément tubulaire 73 du corps 4, vissé dans l'extrémité arrière du fourreau 16.

La construction décrite ci-dessus offre les mêmes avantages qu'une construction classique à quatre paliers du point de vue de l'absorption des charges axiales, mais elle est notablement plus courte et permet donc de réduire sensiblement la longueur totale de la pièce à main. Cette réduction de longueur a le grand avantage d'augmenter la précision de manipulation par l'opérateur, notamment en réduisant l'effet des efforts que le câble 5 exerce sur l'extrémité arrière de l'instrument.

Un autre avantage notable est que, puisque les extrémités adjacentes des deux arbres 11 et 12 sont supportées et centrées par la même bague 62, leur concentricité est assurée sans aucune mesure additionnelle.

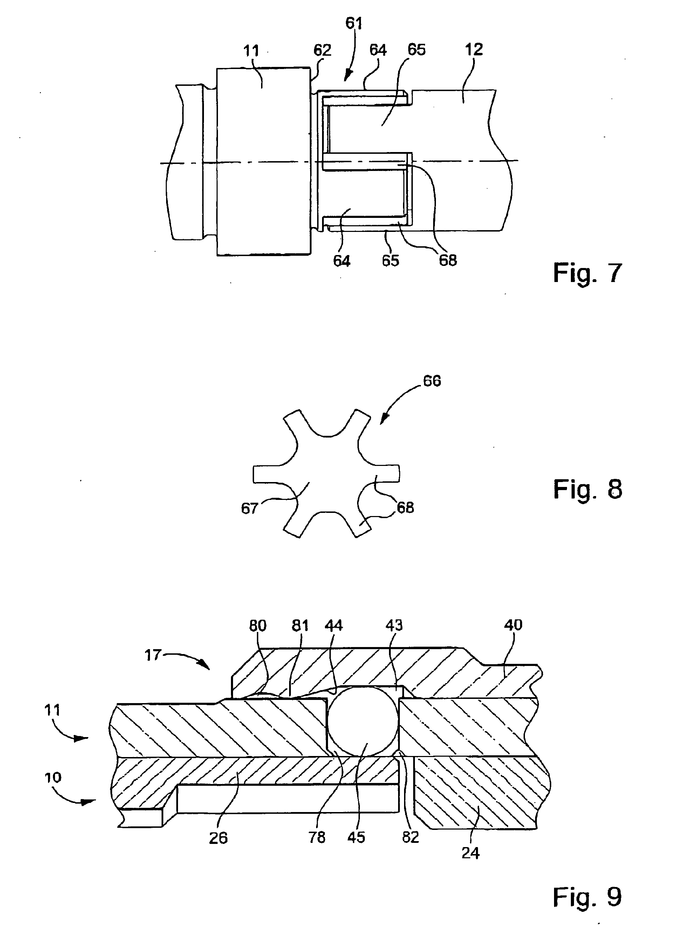

La figure 9 représente un deuxième mode de réalisation du mécanisme de serrage 17, qui permet des simplifications notables par rapport au premier mode de réalisation de la pièce à main. Le mécanisme 17 se distingue de celui décrit plus haut principalement par l'agencement représenté à la figure 9 et par la suppression du ressort central 42 représenté à la figure 2. Le mécanisme de desserrage 18 sert alors de moyen de commande pour le serrage et le desserrage.

Dans la figure 9, on voit que l'extrémité avant du manchon 40 est modifiée simplement par adjonction d'une gorge annulaire 80 à profil en arc de cercle, cette gorge étant séparée de la surface conique 44 par une brève portion de surface cylindrique qui forme une saillie radiale 81 par rapport aux surfaces voisines. Dans ce cas, chaque levier 26 de la pince 10 est de préférence légèrement flexible, ce qui permet au mécanisme de serrage 17 de fonctionner de la manière suivante.

La figure 9 représente la position de desserrage, dans laquelle chaque bille 45 peut aller jusqu'au fond de la gorge 43, de sorte que chaque levier 26 de la pince 10 peut s'écarter jusqu'à s'appliquer contre la surface intérieure de l'arbre creux 11. Les mors 20 de la pince 10 sont alors écartés au maximum et l'on peut introduire ou retirer la queue 3 de l'outil.

Pour serrer ensuite la pince 10, on fait reculer le manchon 40 par rotation du manchon de commande 6 comme dans l'exemple précédent, mais le déplacement axial du manchon 40 est plus grand, car il s'effectue jusqu'à ce que la rainure 80 se place sur les billes 45. Dans un premier temps, le passage de la surface conique 44 sur les billes 45 pousse les leviers 26 vers le centre et les fait fléchir lorsque les mors de la pince rencontrent une résistance suffisante sur la queue de l'outil. Grâce à cette flexion, la saillie cylindrique 81 peut passer par-dessus les billes, puis la gorge 80 va s'encliqueter sur les billes et maintenir en place le manchon coulissant 40 uniquement grâce à la force de réaction des leviers 26 sur les billes 45.

Le desserrage s'effectue sensiblement de la même manière que dans le premier mode de réalisation, par une rotation du manchon de commande 6 (figure 2) qui fait avancer la bague 53 et le manchon jusqu'à la position représentée en figure 9.

La possibilité de supprimer le ressort central 42 représenté à la figure 2 offre des avantages assez importants. D'une part, l'équilibrage de la partie tournante est meilleur, car un tel ressort ne peut jamais être parfaitement centré dans l'alésage qui le contient. Le gain de poids y contribue également. D'autre part, la suppression de ce ressort permet de réduire les forces axiales que le mécanisme de desserrage 18 exerce en direction de l'avant sur le manchon coulissant 40 et, par conséquent, sur l'arbre creux 11. Or ces forces doivent passer ensuite dans les roulements supportant cet arbre, en particulier dans le roulement avant 14 qui est aussi petit que possible et ne devrait pas être soumis à une force axiale trop élevée. Avec l'agencement selon la figure 9, la force axiale maximale impartie à l'arbre 11 lors du desserrage est la force nécessaire pour faire sortir les billes 45 de la gorge 80. Sa valeur peut être prédéterminée aisément par le profil qu'on donne à cette gorge.

Dans la figure 9, on note qu'une collerette 82, ou une série de becs équivalents, est prévue au bord intérieur du trou cylindrique 78 contenant la bille 45, afin de retenir celle-ci lorsque la pince 10 est enlevée. Comme cette collerette n'est pas facile à réaliser, on peut la remplacer par un léger empiétement du canon arrière 24 sur le débouché du trou 78, en raccourcissant légèrement les branches 26 de la pince.

On remarque que le mécanisme de serrage 17 décrit plus haut, que ce soit dans la réalisation selon la figure 2 ou celle de la figure 9, agit seulement sur des surfaces périphériques de la pince 10 et non sur des surfaces ou arêtes de son extrémité. Ceci rend possible diverses variantes de configuration de pince à effet de levier selon les principes exposés ici.

Si l'on considère la figure 2, une de ces variantes consiste à reculer (vers la droite dans le dessin) le canon de guidage arrière 24 et à l'incorporer à la pince 10. les deux extrémités des leviers 26 étant alors rattachées respectivement au canon avant 22 et au canon arrière 24, les leviers étant en outre assez minces pour fléchir radialement entre les canons sous l'action des billes 45. Celles-ci agiraient sur des surfaces d'appui situées sensiblement à mi-longueur des leviers. Les mors 20 pourraient ainsi être situés à la fois près du canon avant 22 et près du canon arrière 24, garantissant ainsi une tenue sans jeu de la queue de l'outil en deux points axialement éloignés.

Une autre variante, dans le même esprit, consisterait à installer dans l'arbre 11 deux pinces telles que la pince 10, disposées tête-bêche et ayant des leviers plus étroits et espacés de façon à pouvoir se placer entre les leviers de l'autre pince sans les toucher. Les mors d'une des pinces se trouveraient à l'avant tandis que ceux de l'autre pince seraient plus en arrière, assurant ainsi une tenue sans jeu de la queue de l'outil en deux points axialement éloignés. Le mécanisme de serrage pourrait comporter deux groupes de billes 45 et deux gorges 43 décalées axialement dans le manchon coulissant 40, pour agir sur les pinces respectives.

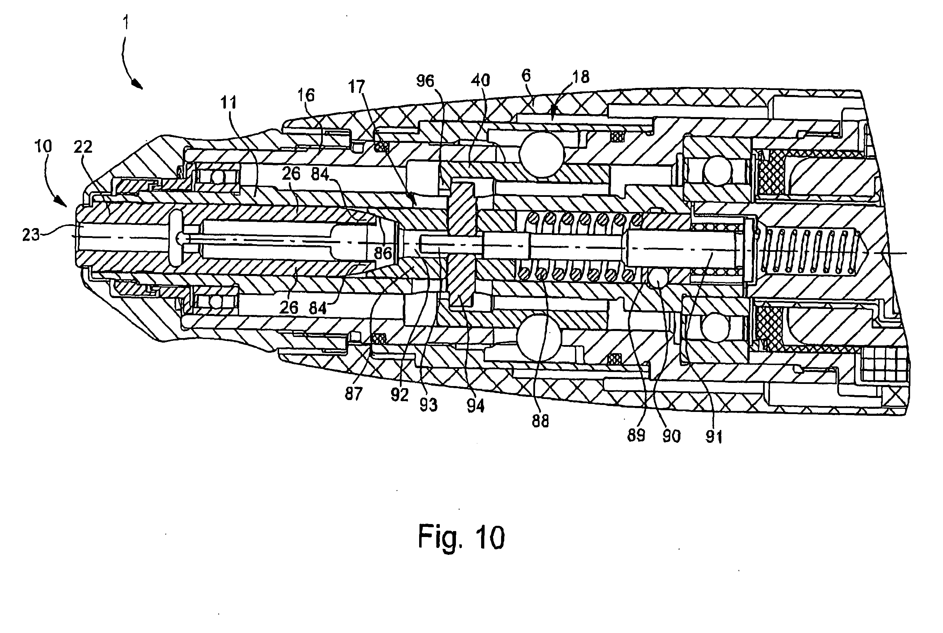

Les figures 10 à 12 illustrent un troisième mode de réalisation de la pièce à main de la figure 1. Les différences par rapport au premier mode de réalisation résident essentiellement dans la construction de la pince et du mécanisme de serrage, si bien qu'on se bornera à décrire ici ces différences, en réutilisant les mêmes numéros de référence pour les pièces semblables.

Dans ce cas, la pince 10 comporte deux leviers 26 diamétralement opposés, ayant à l'arrière des extrémités libres 84 qui sont rétrécies latéralement et qui présentent chacune un arrondi extérieur servant de surface d'appui 85 à une surface conique concave 86 d'un coulisseau 87 monté dans l'arbre creux 11. Ce coulisseau est poussé en permanence vers l'avant par un ressort de compression 88 prenant appui sur une rondelle 89 bloquée dans l'arbre 11 par des billes 90 et un bouchon 91. Le coulisseau 87 comporte un alésage central de guidage 92 destiné à recevoir et guider la queue de l'outil, laquelle bute axialement contre une tige 93 fixée au bouchon 91. Cette tige maintient en outre sur le coulisseau 87 un barreau transversal 94 permettant de repousser le coulisseau vers l'arrière pour relâcher la pince 10 au moyen du mécanisme de desserrage 18. A cet effet, le manchon 40 comporte à l'avant un rebord intérieur 96 capable de s'appuyer contre le barreau 94 quand on fait reculer le manchon 40 par une rotation du manchon de commande 6. Pour le reste, le mécanisme de desserrage 18 peut être semblable à ce qui a été décrit plus haut.

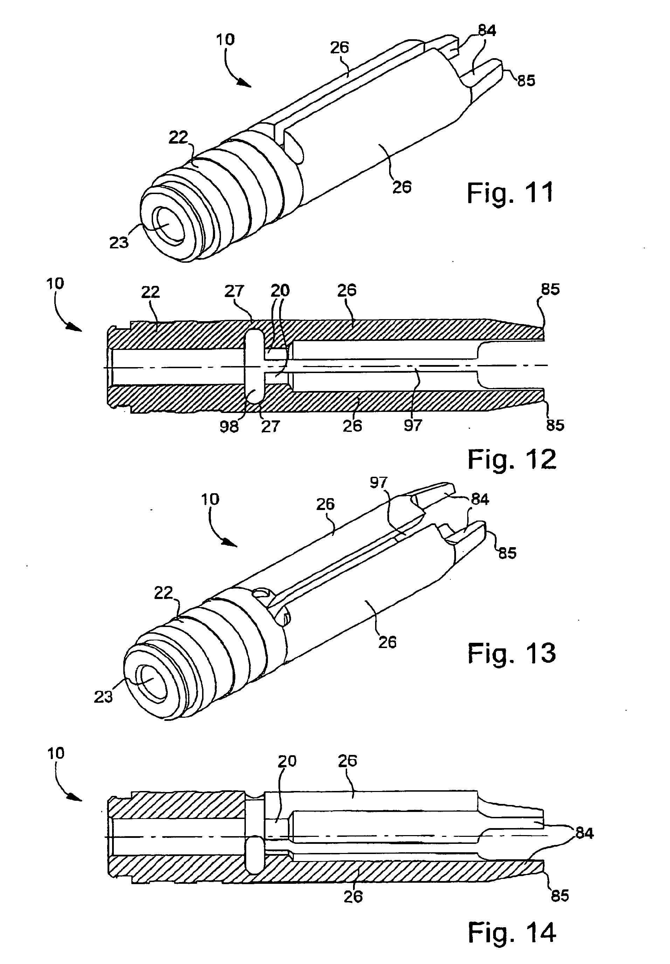

Comme on le voit en particulier dans les figures 11 et 12, la pince 10 à deux mâchoires décrite ci-dessus peut être faite d'une seule pièce, comprenant une partie annulaire servant de manchon de guidage avant 22 et les leviers 26 munis des mors 20. Il suffit de deux fentes longitudinales rectilignes 97, qui peuvent avoir une largeur constante, pour séparer les deux leviers 26 et d'une seule fente transversale 98 pour définir les deux parties flexibles formant les articulations 27 des leviers sur la périphérie de la pince.

Les figures 13 et 14 représentent une pince 10 similaire à celle des figures 10 à 12, mais comportant trois leviers 26 au lieu de deux. Ces leviers sont séparés par des fentes longitudinales 97 réparties à 120 degrés les unes des autres. Ceci montre que la pince utilisée dans la pièce à main selon l'invention peut comporter un nombre quelconque de leviers et de mors, à partir de deux.

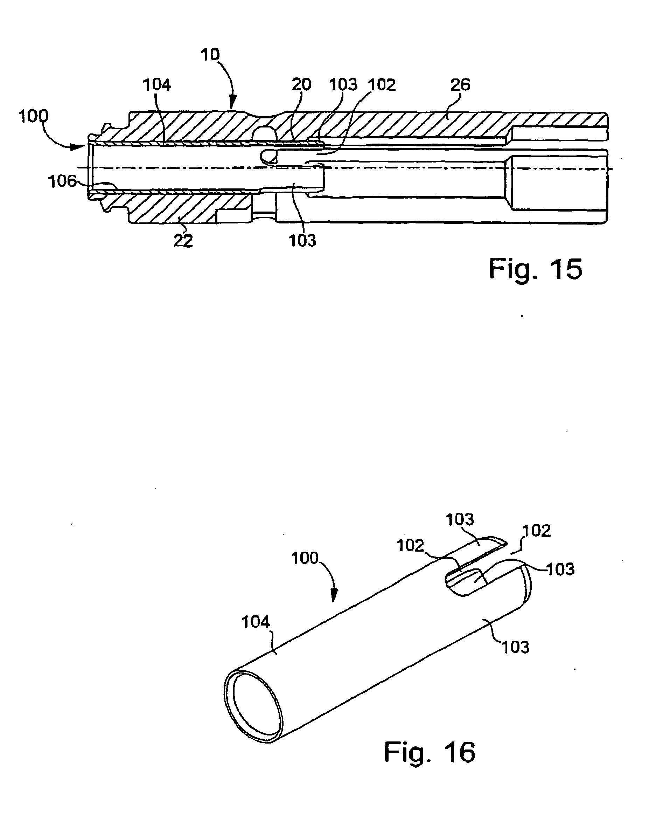

En raison de la très grande force de serrage que peuvent produire les pinces à leviers décrites plus haut, les mors 20 risquent, dans certaines conditions, de détériorer la queue de l'outil ou tout au moins d'y laisser des empreintes. Il est possible de prévenir ce risque en interposant, entre les mors 20 et la queue de l'outil, une douille additionnelle 100 dont un exemple est représenté aux figures 15 et 16. Il s'agit d'une pièce tubulaire cylindrique, de préférence en acier inoxydable trempé, qui pourrait par exemple être fendue sur toute sa longueur pour pouvoir se rétrécir élastiquement sous l'effet de serrage des mors. Dans sa version préférée représentée ici, la douille 100 comporte trois fentes longitudinales 102 qui délimitent trois branches flexibles 103 dirigées vers l'arrière. La partie avant 104 de la douille est entièrement cylindrique et chassée dans la partie annulaire 22 de la pince 10, de sorte que chaque branche flexible 103 se trouve contre l'un des mors 20 en dépassant la surface de serrage du mors de tous côtés. La flexibilité des branches 103 empêche ainsi que les bords des mors laissent une empreinte sur la queue de l'outil. La surface intérieure 106 de la douille 100 est cylindrique et lisse, pour s'appliquer sur la queue de l'outil sans la marquer. Une fois que la douille 100 est en place dans la pince 10, il est avantageux de rectifier sa surface intérieure. On peut ensuite enfiler l'ensemble sur un mandrin de centrage pour rectifier l'extérieur de la pince. Une telle douille additionnelle est utilisable avec n'importe laquelle des pinces 10 décrites plus haut mais, bien entendu, le canal central de la pince doit être élargi dans la mesure qui correspond à l'épaisseur de la paroi de la douille 100.

## Revendications

1. Pièce à main comportant :
- un arbre creux (11) rotatif autour d'un axe longitudinal et monté par des roulements dans un fourreau tubulaire fixe (16),
- une pince (10) montée dans une extrémité avant de l'arbre creux et ayant un canal central (19) destiné à recevoir la queue (3) d'un outil amovible, la pince comportant des branches axiales réparties autour de l'axe et pourvues chacune d'un mors (20) dans le canal central, chaque branche étant rattachée à une partie annulaire (22) de la pince,
- un mécanisme de serrage (17) porté par l'arbre creux (11) et agencé pour exercer une force centripète sur une surface d'appui (46, 85) de chaque branche de la pince pour serrer la queue de l'outil entre les mors de la pince, et
- un mécanisme de desserrage (18) porté au moins en partie par le fourreau fixe (16) et agencé pour agir sur le mécanisme de serrage (17) au moins de façon à relâcher la pince,
**caractérisée en ce que** chaque branche de la pince comprend un levier (26) dont une première extrémité est rattachée à la partie annulaire par une articulation (27) et dont ladite surface d'appui (46, 85) est distante axialement de cette articulation, le mors (20) étant situé axialement plus près de l'articulation (27) que de la surface d'appui (46, 85).

2. Pièce à main selon la revendication 1, **caractérisée en ce que** la partie annulaire (22) et les leviers (26) de la pince sont faits d'une seule pièce, ladite articulation (27) de chaque levier étant une articulation élastique.

3. Pièce à main selon la revendication 1 ou 2, **caractérisée en ce que** la partie annulaire (22) comprend un canon de guidage de la queue d'outil.

4. Pièce à main selon la revendication 1 ou 2, **caractérisée en ce qu**'une douille additionnelle (100) est placée dans le canal central (19) de la pince (10) et comporte des parties flexibles (103) recouvrant les mors (20), en particulier les bords de ceux-ci, du côté de la queue de l'outil.

5. Pièce à main selon l'une des revendications précédentes, **caractérisée en ce que** le mécanisme de serrage (17) comporte un manchon coulissant (40) monté sur l'arbre creux et, pour chaque levier de la pince, un élément de transmission (45) disposé dans un trou radial de l'arbre creux (11) entre ladite surface d'appui (46) du levier et une surface de came intérieure du manchon coulissant (40), la surface de came comportant une surface inclinée axialement telle qu'une surface conique (44).

6. Pièce à main selon la revendication 5, **caractérisée en ce que** ladite surface de came comporte un premier creux (43) pourvu de ladite surface inclinée (44) et en outre un second creux (80) moins profond que le premier et séparé de celui-ci par une partie saillante (81) et **en ce que** les leviers (26) de la pince sont élastiques, de sorte que les seconds creux (80) forment un cran qui maintient en place le manchon coulissant (40) quand lesdits éléments de transmission (45) sont engagés dans les seconds creux par l'élasticité des leviers.

7. Pièce à main selon la revendication 6, **caractérisée en ce que** le manchon coulissant (40) n'est pas sollicité par un ressort, le mécanisme de desserrage (18) étant agencé pour le déplacer en direction axiale dans les deux sens, respectivement pour serrer et desserrer la pince.

8. Pièce à main selon la revendication 5 ou 6, **caractérisée en ce que** lesdits éléments de transmission (45) sont des billes.

9. Pièce à main selon la revendication 4, **caractérisée en ce que** la pince comporte une seconde partie annulaire comprenant un second canon de guidage de la tige d'outil, les leviers de la pince étant flexibles et rattachés à la seconde partie annulaire par leur seconde extrémité, ladite surface d'appui étant située sensiblement à mi-longueur de chaque levier.

10. Pièce à main selon l'une des revendications 1 à 4, **caractérisée en ce que** sur chaque levier (26) de la pince (10), ladite surface d'appui (85) est une surface arrondie à l'extrémité libre du levier et **en ce que** le mécanisme de serrage (17) comporte un coulisseau (87) monté dans l'arbre creux, pourvu d'une surface conique concave (86) du côté de la pince et sollicité axialement par un ressort (88) qui presse sa surface conique contre lesdites surfaces d'appui (85) pour maintenir la pince serrée.

11. Pièce à main selon la revendication 10, **caractérisée en ce que** le coulisseau (87) comporte un alésage de guidage (92) destiné à recevoir la queue d'outil.

12. Pièce à main selon l'une des revendications précédentes, **caractérisée en ce que** le mécanisme de desserrage (18) comporte une bague coulissante (52) disposée dans le fourreau fixe (16) et pourvue d'une gorge annulaire extérieure (55), une douille rotative (50) disposée autour du fourreau fixe (16), liée à un organe manuel de commande (6) et pourvue de rainures axiales intérieures (56), et un jeu de billes (51 ) engagées chacune dans l'une desdites rainures axiales (56), dans une fente hélicoïdale (57) du fourreau fixe (16) et dans ladite gorge annulaire (55), de sorte que la bague coulissante (52) est déplacée axialement par une rotation dans un sens déterminé de la douille rotative (50) et va entraîner le manchon coulissant (40) du mécanisme de serrage de façon à relâcher la pince (10).

13. Pièce à main selon la revendication 12, **caractérisée en ce que** ladite fente hélicoïdale (57) comporte sur un des ses bords, près de son extrémité où se trouve la bille (51) dans la position inactive du mécanisme de desserrage, une languette flexible (58) qui retient la bille par encliquetage dans cette position.

## Claims

1. Handpiece comprising:
- a hollow shaft (11), which is rotatable around a longitudinal axis and mounted by bearings in a fixed tubular sheath (16),
- a clamp (10) mounted in a front end of the hollow shaft and having a central channel (19) intended to receive the shank (3) of a removable tool, the clamp having axial arms arranged around the longitudinal axis, each of which are provided with a gripping jaw (20) in the central channel, and each arm being attached to an annular section (22) of the clamp,
- a tightening mechanism (17) supported by the hollow shaft (11) and arranged to exert a centripetal force on a supporting surface (46, 85) of each arm of the clamp to grip the tool shank between the gripping jaws of the clamp, and
- a release mechanism (18) supported at least partially by the fixed sheath (16) and fitted to act on the tightening mechanism (17) at least so as to loosen the clamp,
**characterized in that** each arm of the clamp comprises a lever (26), a first end of which is attached to the annular section by a joint (27) and said supporting surface (46, 85) of which is axially spaced from this joint, the gripping jaw (20) being located axially closer to the joint (27) than to the supporting surface (46, 85).

2. Handpiece according to claim 1, **characterized in that** the annular section (22) and the levers (26) of the clamp are made from a single piece, said joint (27) of each lever being an elastic joint.

3. Handpiece according to claim 1 or 2, **characterized in that** the annular section (22) comprises a guide tube for the tool shank.

4. Handpiece according to claim 1 or 2, **characterized in that** an additional bushing (100) is positioned in the central channel (19) of the clamp (10) and has flexible parts (103) covering the gripping jaws (20), in particular the edges thereof, in the area of the tool shank.

5. Handpiece according to any of the preceding claims, **characterized in that** the tightening mechanism (17) has a sliding sleeve (40) mounted on the hollow shaft and for each lever of the clamp has a transmission element (45) disposed in a radial hole of the hollow shaft (11) between said supporting surface (46) of the lever and a internal cam surface of the sliding sleeve (40), the cam surface having an axially inclined surface such as a conical surface (44).

6. Handpiece according to claim 5, **characterized in that** said cam surface has a first recess (43) provided for said inclined surface (44) and also a second recess (80), which is not as deep as the first and is separated therefrom by a projecting part (81), and that the levers (26) of the clamp are elastic so that the second recesses (80) form a notch, which holds the sliding sleeve (40) in place when said transmission elements (45) are engaged in the second recesses by the elasticity of the levers.

7. Handpiece according to claim 6, **characterized in that** the sliding sleeve (40) is not biased by a spring, the release mechanism (18) being fitted to shift it axially in both directions respectively for tightening and releasing the clamp.

8. Handpiece according to claim 5 or 6, **characterized in that** said transmission elements (45) are balls.

9. Handpiece according to claim 4, **characterized in that** the clamp has a second annular section comprising a second guide tube for the tool rod, the levers of the clamp being flexible and attached to the second annular section by their second end, said supporting surface being located essentially at mid-length of each lever.

10. Handpiece according to any one of claims 1 to 4, **characterized in that** on each lever (26) of the clamp (10), said supporting surface (85) is a rounded surface at the free end of the lever, and that the tightening mechanism (17) has a slider (87), which is mounted in the hollow shaft and is provided with a concave conical surface (86) in the area of the clamp and is biased axially by a spring (88), which presses its conical surface against said supporting surfaces (85) to keep the clamp tight.

11. Handpiece according to claim 10, **characterized in that** the slider (87) has a guide hole (92) intended to receive the tool shank.

12. Handpiece according to any one of the preceding claims, **characterized in that** the release mechanism (18) has a slide ring (52) disposed in the fixed sheath (16) and provided with an outer annular groove (55), a rotatable bushing (50), which is disposed around the fixed sheath (16), is connected to a manual control means (6) and is provided with axial internal grooves (56), and a set of balls (51), each of which being engaged in one of said axial grooves (56), in a helical slit (57) of the fixed sheath (16) and in said annular groove (55), so that the slide ring (52) is shifted axially by a rotation in a direction determined by the rotatable bushing (50) and will entrain the sliding sleeve (40) of the tightening mechanism so as to loosen the clamp (10).

13. Handpiece according to claim 12, **characterized in that** in an inactive position of the release mechanism, on one of its edges close to its end where the ball (51) is located, said helical slit (57) has a flexible tab (58), which latches the ball in this position.

## Patentansprüche

1. Handstück, das umfasst:
- eine um eine Längsachse rotierende Hohlwelle (11), die durch Lager in einer festen, röhrenförmigen Hülse (16) angebracht ist,
- eine Klemmeinrichtung (10), die in einem Ende vor der Hohlwelle angebracht ist und einen Zentralkanal (19) aufweist, der dazu vorgesehen ist, den Schaft (3) eines abnehmbaren Werkzeugs aufzunehmen, wobei die Klemmeinrichtung axiale Griffe umfasst, die um die Achse herum verteilt sind und jeweils mit einer Backe (20) in dem Zentralkanal versehen sind, wobei jeder Griff mit einem ringförmigen Abschnitt (22) der Klemmeinrichtung verbunden ist,
- eine Spannvorrichtung (17), die durch die Hohlwelle (11) getragen ist und eingerichtet ist, um eine Zentripetalkraft auf eine Stützfläche (46, 85) jedes Griffs der Klemmeinrichtung auszuüben, um den Schaft des Werkzeugs zwischen die Backen der Klemmeinrichtung zu spannen, und
- eine Entspannvorrichtung (18), die zumindest teilweise durch die feste Hülse (16) getragen ist und eingerichtet ist, um die Spannvorrichtung (17) derart zu betätigen, dass die Klemmeinrichtung zumindest gelockert wird,
**dadurch gekennzeichnet, dass** jeder Griff der Klemmeinrichtung einen Hebel (26) umfasst, dessen erstes Ende durch ein Gelenk (27) mit dem ringförmigen Abschnitt verbunden ist und wobei die Stützfläche (46, 85) axial von diesem Gelenk beabstandet liegt, wobei die Backe (20) axial näher an dem Gelenk (27) liegt als die Stützfläche (46, 85).

2. Handstück nach Anspruch 1, **dadurch gekennzeichnet, dass** der ringförmige Abschnitt (22) und die Hebel (26) der Klemmeinrichtung einteilig hergestellt sind, wobei das Gelenk (27) jedes Hebels ein elastisches Gelenk ist.

3. Handstück nach Anspruch 1 oder 2, **dadurch gekennzeichnet, dass** der ringförmige Abschnitt (22) ein Führungsrohr des Werkzeugschafts umfasst.

4. Handstück nach Anspruch 1 oder 2, **dadurch gekennzeichnet, dass** eine zusätzliche Buchse (100) in dem Zentralkanal (19) der Klemmeinrichtung (10) angeordnet ist und biegsame Abschnitte (103) umfasst, die die Backen (20), insbesondere deren Ränder, auf der Seite des Schafts des Werkzeugs abdecken.

5. Handstück nach einem der vorhergehenden Ansprüche, **dadurch gekennzeichnet, dass** die Spannvorrichtung (17) eine Schiebemuffe (40), die auf der Hohlwelle angebracht ist, und für jeden Hebel der Klemmeinrichtung ein Kraftübertragungselement (45) umfasst, das in einer Radialbohrung der Hohlwelle (11) zwischen der Stützfläche (46) des Hebels und einer inneren Nockenfläche der Schiebemuffe (40) angeordnet ist, wobei die Nockenfläche eine axial geneigte Fläche wie z. B. eine kegelförmige Fläche (44) umfasst.

6. Handstück nach Anspruch 5, **dadurch gekennzeichnet, dass** die Nockenfläche eine erste Ausnehmung (43), die mit der geneigten Fläche (44) versehen ist, und überdies eine zweite Ausnehmung (80) umfasst, die weniger tief als die erste ist und durch einen vorstehenden Abschnitt (81) von ihr getrennt ist, und **dadurch**, dass die Hebel (26) der Klemmeinrichtung elastisch sind, sodass die zweiten Ausnehmungen (80) eine Kerbe bilden, die die Schiebemuffe (40) in ihrer Position hält, wenn die Kraftübertragungselemente (45) durch die Elastizität der Hebel in den zweiten Ausnehmungen in Eingriff stehen.

7. Handstück nach Anspruch 6, **dadurch gekennzeichnet, dass** die Schiebemuffe (40) nicht durch eine Feder gespannt ist, wobei die Entspannvorrichtung (18) eingerichtet ist, um sie in beiden Richtungen in Axialrichtung zu verschieben und die Klemmeinrichtung zu spannen bzw. zu lösen.

8. Handstück nach Anspruch 5 oder 6, **dadurch gekennzeichnet, dass** die Kraftübertragungselemente (45) Kugeln sind.

9. Handstück nach Anspruch 4, **dadurch gekennzeichnet, dass** die Klemmeinrichtung einen zweiten ringförmigen Abschnitt aufweist, der ein zweites Führungsrohr des Werkzeugschafts umfasst, wobei die Hebel der Klemmeinrichtung biegsam sind und über ihr zweites Ende mit dem zweiten ringförmigen Abschnitt verbunden sind, wobei die Stützfläche sich im Wesentlichen auf halber Länge jedes Hebels befindet.

10. Handstück nach einem der Ansprüche 1 bis 4, **dadurch gekennzeichnet, dass** die Stützfläche (85) auf jedem Hebel (26) der Klemmeinrichtung (10) eine an dem freien Ende des Hebels gerundete Fläche ist, und **dadurch**, dass die Spannvorrichtung (17) eine in der Hohlwelle angebrachte Schiebevorrichtung (87) umfasst, die auf der Seite der Klemmeinrichtung mit einer konkaven, kegelförmigen Fläche (86) versehen ist und axial durch eine Feder (88) gespannt ist, die seine kegelförmige Fläche gegen die Stützflächen (85) presst, um die Klemmeinrichtung gespannt zu halten.

11. Handstück nach Anspruch 10, **dadurch gekennzeichnet, dass** der Schiebevorrichtung (87) eine Führungsbohrung (92) umfasst, die zum Aufnehmen des Werkzeugschafts bestimmt ist.

12. Handstück nach einem der vorhergehenden Ansprüche, **dadurch gekennzeichnet, dass** die Entspannvorrichtung (18) einen Gleitring (52), der in der festen Hülse (16) angeordnet ist und mit einer äußeren ringförmigen Auskehlung (55) versehen ist, eine rotierende Buchse (50), die um die feste Hülse (16) herum angeordnet ist, welche mit einem Handbedienteil (6) verbunden ist und mit inneren axialen Nuten (56) versehen ist, und einen Satz von Kugeln (51) umfasst, die jeweils in einer der axialen Nuten (56), in einem schraubenförmigen Schlitz (57) der festen Hülse (16) und in der ringförmigen Auskehlung (55) in Eingriff stehen, sodass der Gleitring (52) durch eine Rotation der rotierenden Buchse (50) in eine bestimmte Richtung axial verschoben wird und die Schiebemuffe (40) der Spannvorrichtung derart mitnimmt, dass die Klemmeinrichtung (10) gelockert wird.

13. Handstück nach Anspruch 12, **dadurch gekennzeichnet, dass** der schraubenförmige Schlitz (57) auf einem seiner Ränder in der Nähe seines Endes, an dem sich die Kugel (51) in der nicht aktiven Position der Entspannvorrichtung befindet, eine biegsame Zunge (58) umfasst, die die Kugel durch Einrasten in dieser Position hält.
